(19) Europäisches Patentamt
European Patent Office
Office européen des brevets

(11) Veröffentlichungsnummer: **0 293 742 B1**

(12) **EUROPÄISCHE PATENTSCHRIFT**

(45) Veröffentlichungstag der Patentschrift: **22.07.92**

(51) Int. Cl.5: **C07D 233/92**, C07D 233/94, C07D 473/38, C07D 401/12

(21) Anmeldenummer: **88108359.6**

(22) Anmeldetag: **26.05.88**

(54) **Verfahren zur Herstellung von 4-Nitro-5-imidazolylethern und -thioethern.**

(30) Priorität: **05.06.87 DE 3718898**
**03.09.87 DE 3729406**

(43) Veröffentlichungstag der Anmeldung:
**07.12.88 Patentblatt 88/49**

(45) Bekanntmachung des Hinweises auf die Patenterteilung:
**22.07.92 Patentblatt 92/30**

(84) Benannte Vertragsstaaten:
**CH DE FR GB IT LI**

(56) Entgegenhaltungen:
**DD-A- 220 961**
**US-A- 3 056 785**
**US-A- 3 862 061**

**CHEMICAL ABSTRACTS, Band 107, Nr. 23, 7. Dezember 1987, Columbus, Ohio, US; BALONIAK, S.; MROCZKIEWICZ, A.; HOFFMANN, B.; WIKA, F.; LUKOWSKI, A.: "Preparation of [(1,2-dimethyl-4-nitro-5-imidazolyl)thio]purine" Seite 564, Spalte 2, Zusammenfassung-Nr. 217 370h**

**CHEMICAL ABSTRACTS, Band 107, Nr. 8, 24. August 1987, Columbus, Ohio, US; SUWINSKI, J.; SALWINSKA, E.; WATRAS, J.; WIDEL, M.: "Nitroimidazoles. VIII. Synthesis of 1-(2-hydroxy-3-methoxypropyl)- und 1-(2,3-dihydroxypropyl)-4(5)-nitro-5(4)-substituted imidazoles" Seite 695, Spalte 2, Zusammenfassung-Nr. 77 701q**

**CHEMICAL ABSTRACTS, Band 85, Nr. 1, 5. Juli 1976, Columbus, Ohio, US; ICHINO, M.; SATO, M.; KANAI, T.; NAKAMURA, T.: "4-Mercaptopyrazolo[3,4-d]pyrimidine derivative" Seite 459, Spalte 2, Zusammenfassung-Nr. 5 680m**

(73) Patentinhaber: **BASF Aktiengesellschaft**
**Carl-Bosch-Strasse 38**
**W-6700 Ludwigshafen(DE)**

(72) Erfinder: **Koehler, Hermann, Dr.**
**Roentgenstrasse 7**
**W-6711 Beindersheim(DE)**
Erfinder: **Dockner, Toni, Dr.**
**Grossgasse 6**
**W-6701 Meckenheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft ein Verfahren zur Herstellung von 4-Nitro-4-imidazolylethern und -thioethern.

Aus P.M. Kochergin et al., Chemistry of Heterocyclic Compounds 1971, 648 und V.P. Arya et al., Indian Journal of Chemistry 21 B, 1115 (1982) ist bekannt, daß Ether der Formel I mit $R^1$ = $CH_3$ und $R^2$ = H durch Umsetzung von 1-Methyl-4-nitro-5-chlorimidazol mit Sauerstoffverbindungen erhalten werden.

Das 1-Methyl-4-nitro-5-chlorimidazol ist nur in schlechter Ausbeute durch Umsetzung von N, N′-Dimethyloxalsäurediamid mit Phosphorpentachlorid und nachfolgende Nitrierung des gebildeten 1-Methyl-5-chlorimidazols zugänglich (O. Wallach, Liebigs Annalen der Chemie 184, 51 (1877)).

Aus L. L. Bennett, Jr.: H. T. Baker, J. Amer. Chem. Soc. 79, 2188 (1957) ist bekannt, daß Thioether der Formel I mit $R^1$ = $CH_3$ und $R^2$ = H durch Umsetzung von 1-Methyl-4-nitro-5-chlor-imidazol mit Mercaptanen in stark alkalischer Lösung erhalten werden. Führt man die Umsetzung dieses Chlorimidazols oder des 2-Methylhomologen in Gegenwart 20 %iger Natriumcarbonatlösung durch, so können unterschiedliche alkyl-, aryl- oder heterocyclisch substituierte Thiole verwendet werden, wie aus Chemical Abstracts 96, 104146a, 1981 hervorgeht.

Weiterhin können diese Verbindungen durch Umsetzung des Natrium-Thiolates von 1-Methyl-4-nitro-5-mercapto-imidazol mit Alkylhalogeniden hergestellt werden (P. M. Kochergin, I. S. Shmidt, Med. Prom. SSSR 19, 6 (1965), vgl. CA 64 735 a (1966)), wobei das Thiolat seinerseits aus dem 1-Methyl-4-nitro-5-chlorimidazol (L. L. Bennett, Jr.; H. T. Baker, loc. cit) erhalten werden kann.

Ein Nachteil der beschriebenen Verfahren ist die geringe Anwendungsbreite.

Der Erfindung lag daher die Aufgabe zugrunde, ein neues und verbessertes Verfahren zur Herstellung von 4-Nitro-5-imidazolylethern und -thioethern der Formel I zu finden und den Nachteilen der bekannten Verfahren abzuhelfen.

Demgemäß wurde ein neues und verbessertes Verfahren zur Herstellung von 4-Nitro-5-imidazolylethern und -thioethern der Formel I

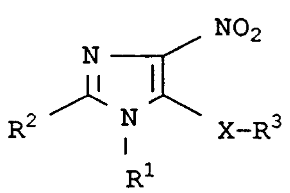

(I),

in der die Reste

- $R^1$ $C_1$- bis $C_{18}$-Alkyl, $C_2$- bis $C_8$-Alkoxyalkyl, $C_5$- bis $C_8$-Cycloalkyl, Aryl, $C_7$- bis $C_{12}$-Arylalkyl, $C_7$- bis $C_{12}$-Alkylaryl,
- $R^2$ Wasserstoff, $C_1$- bis $C_{18}$-Alkyl, $C_5$- bis $C_8$-Cycloalkyl, Aryl, $C_7$- bis $C_{12}$-Arylalkyl, $C_7$- bis Alkylaryl,
- $R^3$ $C_1$- bis $C_{18}$-Alkyl, $C_2$- bis $C_8$-Alkoxyalkyl, $C_5$- bis $C_8$-Cycloalkyl, Aryl, $C_7$- bis $C_{12}$-Arylalkyl, $C_7$- bis $C_{12}$-Alkylaryl oder 3-Pyridyl, 2-Pyridyl, 2-Pyrimidyl, 8-Chinolyl, 2-Imidazolyl, 2-Thiazolyl, 6-Purimidyl, 3-Tetrahydropyranyl und 2-Thiazolinyl, ein Gegenion des entsprechenden Alkoholates oder für den Fall, daß X = Schwefel ist $R^3$ = Wasserstoff, und
- X Sauerstoff oder Schwefel

bedeuten, wobei die genannten Aryl Reste $C_1$- bis $C_4$-Alkyl, $C_1$- bis $C_4$-Alkoxy, Halogen, Halogenalkyl und/oder $C_1$- bis $C_4$-Alkoxymethyl, die organischen Reste $R^1$ und $R^2$ $C_1$- bis $C_4$-Alkyl, $C_1$- bis $C_4$-Alkoxy und/oder Halogen, und der Rest $R^3$ $C_1$- bis $C_6$-Alkyl, $C_1$- bis $C_6$-Halogenalkyl, $C_1$- bis $C_6$-Alkoxy, $C_1$- bis $C_6$-Dialkylamino und/oder Halogen Substituenten tragen können, gefunden, welches dadurch gekennzeichnet ist, daß man Dinitroimidazole der Formel II

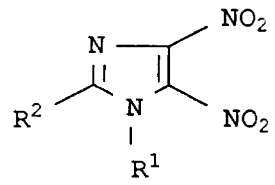

(II),

mit einem Alkohol oder Thiol der Formel III

$R^3$-XH (III),

in einem Lösungs- oder Verdünnungsmittel bei Temperaturen von 0 bis 150°C und einem pH-Wert von 4 bis 16 umsetzt.

Der Erfolg des erfindungsgemäßen Verfahrens ist überraschend insbesondere im Hinblick darauf, daß selektiv nur die Nitrogruppe in 5-Stellung des Imidazols II ausgetauscht wird.

Die Herstellung von Verbindungen der Formel II ist beschrieben (S. S. Novikov et al, Chem. Heterocyclic Compds. 1970, 465, vgl. CA 73, 66491 z (1970)). Nach dem dort angegebenen Verfahren ist eine große Zahl von verschieden substituierten Verbindungn der Formel II zugänglich.

So kann der Rest $R^1$ Alkyl, Alkoxyalkyl, Cycloalkyl, Aryl, Arylalkyl oder Alkylaryl bezeichnen. Alkylreste sind z.B. verzweigte oder unverzweigte Reste mit 1 bis 18, insbesondere 1 bis 8 Kohlenstoffatomen wie Methyl, Ethyl, Propyl, Isopropyl, n-Butyl, sek.-Butyl, tert.-Butyl, Pentyl, Hexyl, Heptyl oder Octyl. Alkoxyalkylreste haben z.B. insgesamt 2 bis 8 Kohlenstoffatome wie $C_1$-$C_4$-Alkoxymethyl, Methoxyethyl oder Ethoxyethyl. Cycloalkylreste sind z.B. $C_5$- bis $C_8$-Cycloalkylreste, insbesondere Cyclopentyl- oder Cyclohexyl. Arylreste sind z.B. Phenyl, Naphthyl, ggf. substituiert durch inerte Gruppen wie $C_1$-$C_4$-Alkyl, $C_1$-$C_4$-Alkoxy, Halogen wie Fluor, Chlor oder Brom, Halogenalkyl wie Trifluormethyl, Difluormethyl oder Dichlormethyl oder $C_1$-$C_4$-Alkoxymethyl. Arylalkyl- oder Alkylarylreste sind insbesondere solche mit 7 bis 12 Kohlenstoffatomen wie Tolyl, Xylyl, Phenylethyl oder Benzyl, die ebenso wie die Arylreste substituiert sein können.

Der Rest $R^2$ kann die Bedeutung von $R^1$ haben und darüber hinaus für Wasserstoff stehen. Die organischen Reste $R^1$ und/oder $R^2$ können noch unter den Reaktionsbedinungen inerte Substituenten tragen wie $C_1$-$C_4$-Alkyl- oder Alkoxygruppen oder Halogen die Fluor, Chlor oder Brom. Der Rest $R^2$ steht bevorzugt für Wasserstoff, $c_1$-$C_4$-Alkyl, Alkoxymethyl wie Methyl, Ethyl, Isopropyl, Methoxymethyl, Phenylethyl und Benzyl.

Die Alkohole und Thiole der Formel III sind bekannt und können nach literaturüblichen Verfahren hergestellt werden.

So kann $R^3$ Wasserstoff, Alkyl, Alkoxyalkyl, Cycloalkyl, Aryl, Arylalkyl oder Alkylaryl bezeichnen, d.h. die für $R^1$ genannte Bedeutung haben, wobei die genannten Reste noch durch inerte Gruppen wie niedermolekulare Alkyl-, Halogenalkyl-, Alkoxy- oder Dialkylaminogruppen oder durch Halogen wie Fluor, Chlor oder Brom substituiert sein können. Vorteilhaft enthalten die inerten Substituenten 1 bis 6 Kohlenstoffatome. $R^3$ steht z.B. für Wasserstoff, $C_1$-$C_4$-Alkyl wie Methyl, Ethyl, Propyl, Isopropyl oder Butyl, Aryl oder Aralkyl wie Phenyl, 1-Naphthyl, 2-Naphthyl, 4-Chlorphenyl, 2,4-Dichlorphenyl, 2-Bromphenyl, 4-Methoxyphenyl, 3-(Trifluormethyl)-phenyl, 3-(Difluormethyl)-phenyl, Benzyl oder 4-Chlorbenzyl.

Weiterhin kann $R^3$ einen heterocyclischen oder heteroaromatischen Rest darstellen, wobei als Heteroatome insbesondere Stickstoff neben Sauerstoff und Schwefel in Betracht kommen. Die heteroaromatischen Reste haben vorteilhaft 5 oder 6 Ringglieder mit 1 bis 2 Heteroatomen und können einen weiteren 5- oder 6-Ring, der z.B. aromatisch oder heteroaromatisch ist, ankondensiert haben. Beispielsweise seien folgende Reste aufgeführt: 3-Pyridyl, 2-Pyridyl, 2-Pyrimidyl, 8-Chinolyl, 2-Imidazolyl, 2-Thiazolyl oder 6-Purinyl. Heterocyclische Reste sind z.B. gesättigte oder Doppelbindungen enthaltende heterocyclische Reste mit 1 oder 2 Heteroatomen wie 3-Tetrahydropyridyl oder 2-Thiazolinyl.

Der Rest $R^3$ kann bei Verwendung starker Basen und bei Umsetzung in alkalischem Medium auch für das Gegenion des entsprechenden Alkoholates oder Thiolates stehen. Als Gegenion kommen je nach verwendeter Base z.B. Alkalimetall- oder Erdalkalimetalllionen oder Ammoniumionen in Betracht, insbesondere Natrium, Kalium, Calcium, Ammonium oder Trialkylammonium.

Nach dem erfindungsgemäßen Verfahren können 4-Nitro-5-imidazolylether und -thioether der Struktur I in einfacher Weise dadurch erhalten werden, daß man Dinitroimidazole II mit den Verbindungen III oder im Fall von $R^3$ = H mit Schwefelwasserstoff in einem Lösungs- oder Verdünnungsmittel bei einem pH-Wert

von 4 oder darüber, z.B. 4 bis 16, insbesondere 6 bis 14, besonders vorteilhaft 8 bis 14, miteinander umsetzt.

Als Lösungs- oder Verdünnungsmittel kommen insbesondere Wasser und/oder mit Wasser mischbare inerte organische Lösungsmittel in Betracht. Geeignete organische Lösungsmittel sind z.B. niedermolekulare Alkohole, Glykole und Glykolderivate wie Ethylenglykol, Methyl- oder Ethylglykol sowie Säureamide wie Dimethylformamid. Bevorzugte Lösungsmittel sind Wasser und/oder $C_1$-$C_4$-Alkohole, z.B. Methanol, Ethanol, Propanol, Isopropanol, Butanol und Isobutanol.

Der pH-Wert des Reaktionsgemisches wird im allgemeinen durch Zugabe einer Base eingestellt. Als Basen kommen beispielsweise Hydroxide. Carbonate oder Hydrogencarbonate der Alkali- oder Erdalkalimetalle, Ammoniak oder organische Stickstoffverbindungen wie tertiäre Amine, z.B. Trimethyl-, Triethyl-, Tripropyl-, Tributylamin, N,N-Dimethylanilin oder Pyridin in Betracht. Bevorzugt können Carbonate wie Kalium- oder Natriumcarbonat, Trialkylamine oder Ammoniak verwendet werden. Zur Herstellung von Thiolaten wird man insbesondere starke Basen wie Natrium oder Kaliumhydroxid, Calciumhydroxid oder Amine wählen.

Die Basenmenge ist nicht besonders kritisch, es können z.B. 0,1 bis 100 mol, vorteilhaft 1 bis 20 mol je Mol Dinitroimidazol II verwendet werden.

Das Molverhältnis der Verbindung III, bezogen auf das Dinitroimdazol II beträft zweckmäßigerweise 1 bis 5, vorteilhaft 1 bis 3, besonders vorteilhaft 1 bis 1,2.

Die Reaktion kann unter äußerst milden Bedingungen durchgeführt werden. Temperaturen von 0 bis 150 ° C sind ausreichend. Bevorzugt wird die Umsetzung bei Temperaturen von 20 bis 80 ° C vorgenommen.

Die Reaktion wird üblicherweise bei Normaldruck kontinuierlich oder diskontinuierlich in den dafür geeigneten Apparaturen durchgeführt. Im Falle von flüchtigen Verbindungen III, z.B. Methylmercaptan, kann es vorteilhaft sein, unter leichtem Überdruck in einer geschlossenen Apparatur zu arbeiten.

Die Isolierung und Reinigung der Produkte erfolgt in an sich bekannter Weise z.B. durch Abfiltrieren der festen Produkte und Umkristallisieren aus geeigneten Lösungsmitteln. Im Fall von $R^3$ = H wird man das Endprodukt aus dem entsprechenden Alkoholat oder Thiolat durch Ansäuern freisetzen. Häufig werden aber die Salze selbst, z.B. die Natrium- oder Ammoniumsalze der 4-Nitro-5-imidazolylether und -thioether direkt für weitere Umsetzungen verwendet.

Nach der erfindungsgemäßen Verfahrensweise fallen die 4-Nitro-5-imidazolylether und -thioether I in guten bis sehr guten Ausbeuten an.

4-Nitro-5-imidazolylether und -thioether I sind als Zwischenprodukte zur Herstellung von Farbstoffen und Pflanzenschutzmitteln von Interesse. Vorzugsweise können die Imidazole I zur Herstellung von pharmakologisch wirksamen Verbindungen verwendet werden oder dienen selbst als Wirkstoffe. Beispielsweise ist das 6-(1-Methyl-4-nitro-5-imidazolyl)-mercaptopurin ein wirksames Immunsuppressivum (siehe US-PS 3 056 785).

Die folgenden Beispiele sollen die Erfindung näher erläutern, ohne sie einzuschränken.

Beispiel 1

Herstellung von 1-Methyl-4-nitro-5-mercaptophenylimidazol

Zu einem Gemisch aus 17,2 g (0,1 mol) 1-Methyl-4,5-dinitroimidazol, 75 ml 25 %igem $NH_3$ und 75 ml Wasser wurden unter Rühren 13,2 g (0,12 mol) Thiophenol gegeben, wobei die Temperatur der Lösung von 22 ° C auf 38 ° C anstieg. Man rührte noch 0,5 Stunden nach, filtrierte dann die ausgefallenen Kristalle ab und kristallisierte aus Isopropanol um.

Ausbeute: 21, 9 g (93 %) 1-Methyl-4-nitro-5-mercaptophenylimidazol, Smp. 73 bis 75 ° C.

Beispiele 2 bis 9

Herstellung verschieden substituierter 1-Methyl-4-nitroimidazole

Entsprechend Beispiel 1 wurden die in nachfolgender Tabelle A aufgeführten Beispiele durchgeführt.

Tabelle A

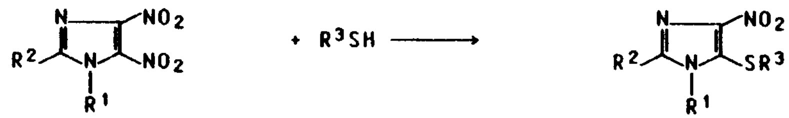


| Beispiel | $R^1$ | $R^2$ | $R^3$ | Ausbeute % bezogen auf II | Smp. °C | Elementaranalyse C:H:N:S in % |
|---|---|---|---|---|---|---|
| 2 | $CH_3$ | H | $CH_3-CH_2-CH_2-$ | 65 | 43- 44 | - |
| 3 | $CH_3$ | H | $CH_3-(CH_2)_{11}-$ | 73 | 86- 88 | Gef.: 58,4:8,7:13,0:9,5<br>Ber.: 58,7:8,9:12,8:9,8 |
| 4 | $CH_3$ | H | 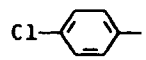 | 78 | 128-129 | Gef.: 44,6:3,1:15,3:11,5; Cl:13,2<br>Ber.: 44,5:3,0:15,6:11,9; Cl:13,1 |
| 5 | $CH_3$ | H | 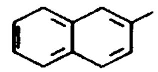 | 67 | 154-156 | Gef.: 60,1:3,9:13,7:12,0<br>Ber.: 58,9:3,9:14,7:11,2 |
| 6 | $CH_3$ | H | 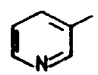 | 89 | 95- 97 | Gef.: 44,7:3,1:24,9:12,1<br>Ber.: 45,8:3,4:23,7:13,6 |
| 7 | $CH_3$ | H | 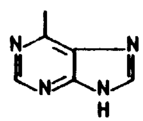 | 96 | 251-253 | - |
| 8 | $CH_3$ | $CH_3$ | 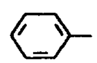 | 76 | 89- 92 | Gef.: 52,8:4,6:17,1:12,8<br>Ber.: 52,9:4,4:16,8:12,9 |
| 9 | $C_6H_5-CH_2$ | $CH_3$ | 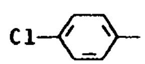 | 90 | 123-125 | Gef.: 56,8:4,0:11,6:8,8; Cl:10,0<br>Ber.: 56,7:3,9:11,7:8,9; Cl: 9,9 |

Beispiel 10

Herstellung von 1-Methyl-4-nitro-5-mercaptophenylimidazol

Zu einer gerührten Suspension von 17,2 g (0,1 mol) 1-Methyl-4,5-dinitroimidazol in 65 ml Wasser wurden 69 g $K_2CO_3$ und dann 12,1 g (0,11 mol) Thiophenol gegeben, wobei die Temperatur der Lösung von 22° C auf 40° C anstieg. Man rührte noch 1 Stunde bei dieser Temperatur nach, filtrierte dann die ausgefallenen Kristalle ab und kristallisierte aus Isopropanol um.

Ausbeute: 19,3 g (82 %) 1-Methyl-4-nitro-5-mercaptophenylimidazol, Smp. 73 bis 76° C.

Beispiel 11

Herstellung von 1-Methyl-4-nitro-5-mercaptophenylimidazol

Zu einer gerührten Suspension von 17,2 g (0,1 mol) 1-Methyl-4,5-dinitroimidazol in 20,2 g Triethylamin und 75 ml Ethanol wurden 12,1 g (0,11 mol) Thiophenol gegeben, wobei die Temperatur der Lösung von 22° C auf 50° C anstieg. Man rührte noch 0,5 Stunden bei dieser Temperatur nach und versetzte dann die Lösung mit 150 ml Isopropanol. Die ausgefallenen Kristalle werden abgesaugt und aus Isopropanol umkristallisiert.

Ausbeute: 20,9 g (89 %), Smp. 73 bis 76° C.

Beispiel 12

Herstellung des Ammoniumsalzes von 1-Methyl-4-nitro-5-mercaptoimidazol

Durch ein Gemisch aus 17,2 g (0,1 mol) 1-Methyl-4,5-dinitroimidazol, 75 ml 25 %iges $NH_3$ und 75 ml Wasser leitete man für 0,25 Stunden unter Rühren einen schwachen $H_2S$-Strom, wobei die Temperatur der Lösung von 21° C auf 55° C anstieg. Man rührte noch 1 Stunde nach, filtrierte dann die ausgefallenen Kristalle ab und kristallisierte aus Methanol um.

Ausbeute: 16,8 g (95 %) Ammoniumsalz von 1-Methyl-4-nitro-5-mercaptoimidazol, Smp. 195 bis 198° C.

Beispiel 13

Herstellung von 1-Methyl-4-nitro-5-phenoxyimidazol

Eine Mischung aus 17,2 g (0,1 mol) 1-Methyl-4,5-dinitroimidazol, 11,2 g (0,12 mol) Phenol und 4,4 g (0,11 mol) NaOH in 200 ml Wasser wurde 1 h bei 50° C gerührt. Dann wurde die Lösung mit weiteren 300 ml Wasser verdünnt und abgekühlt. Die ausgefallenen Kristalle wurden abgesaugt und aus Wasser umkristallisiert. Ausbeute: 21,0 g (96 %) 1-Methyl-4-nitro-5-phenoxyimidazol; Smp. 116-118° C.

| Elementaranalyse: | | | | |
|---|---|---|---|---|
| Ber.: | C 54,8 % | H 4,1 % | N 19,2 % | O 21,9 % |
| Gef.: | C 54,2 % | H 4,0 % | N 18,9 % | O 22,2 % |

Beispiele 14 bis 23

Entsprechend Beispiel 13 wurden die in der nachfolgenden Tabelle 8 gekennzeichneten Verbindungen hergestellt:

Tabelle B

| Beispiel | $R^1$ | $R^2$ | $R^3$ | Ausbeute | Smp. (°C) | Elementaranalyse |
|---|---|---|---|---|---|---|
| 14 | $CH_3$- | H- | 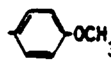 | 97 % | 119 - 121 | Ber: C 53,0; H 4,5; N 16,9; O 25,7<br>Gef: C 52,9; H 4,8; N 17,1; O 25,8 |
| 15 | $CH_3$- | H- | 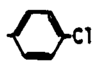 | 97 % | 116 - 118 | Ber: C 47,4; H 3,2; N 16,6; O 18,9; Cl 14,0<br>Gef: C 47,2; H 3,5; N 16,8; O 18,7; Cl 13,7 |
| 16 | $CH_3$- | H- | 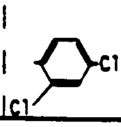 | 67 % | 168 - 171 | Ber: C 41,7; H 2,5; N 14,6; O 16,7; Cl 24,6<br>Gef: C 41,3; H 2,7; N 15,0; O 16,4; Cl 23,7 |
| 17 | $CH_3$- | H- | 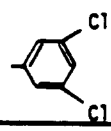 | 69 % | 206 - 207 | Ber: C 41,7; H 2,5; N 14,6; O 16,7; Cl 24,6<br>Gef: C 41,3; H 2,6; N 14,7; O 16,4; Cl 24,3 |
| 18 | $CH_3$- | H- | 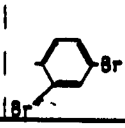 | 67 % | 194 - 196 | Ber: C 31,9; H 1,9; N 11,1; O 12,7; Br 42,2<br>Gef: C 32,0; H 1,9; N 11,3; O 12,8; Cl 42,4 |
| 19 | $CH_3$- | H- | 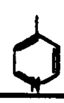 | 75 % | 241 - 243 | Ber: C 49,1; H 3,7; N 25,4; O 21,8<br>Gef: C 48,9; H 4,1; N 25,9; O 21,6 |
| 20 | $CH_3$- | H- | 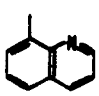 | 97 % | 173 - 175 | Ber: C 57,8; H 3,7; N 20,7; O 17,8<br>Gef: C 57,6; H 4,2; N 20,9; O 17,5 |
| 21 | $CH_3$- | $CH_3$ | 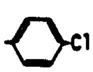 | 92 % | 181 - 182 | Ber: C 49,4; H 3,8; N 15,7; O 17,9; Cl 13,2<br>Gef: C 49,2; H 3,8; N 15,6; O 17,5; Cl 13,8 |
| 22 | $CH_3$- | $CH_3$- | 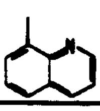 | 90 % | 181 - 182 | Ber: C 59,2; H 4,3; N 19,7; O 16,9<br>Gef: C 58,9; H 4,3; N 19,3; O 17,0 |
| 23 | $CH_2$- 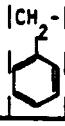 | $CH_3$- | 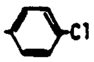 | 85 % | 164 - 165 | Ber: C 59,4; H 4,1; N 12,2; O 14,0; Cl 10,3<br>Gef: C 59,4; H 4,3; N 12,1; O 14,4; Cl 10,6 |

Beispiel 24

Herstellung von 1-Methyl-4-nitro-5-methoxyimidazol

Eine Lösung von 17,2 g (0,1 mol) 1-Methyl-4,5-dinitroimidazol und 6,0 g (0,11 mol) Natriummethylat in 200 ml Methanol wurde 1 h bei 60 °C gerührt. Dann wurde die Lösung eingedampft und der Rückstand aus Wasser umkristallisiert.

Ausbeute: 10,0 g (64 %) 1-Methyl-4-nitro-5-methoxyimidazol, Smp. 140-141 °C (Literatur: 134-135 °C).

Beispiel 25

Herstellung von 1-Methyl-4-nitro-5-phenoxyimidazol

Eine Lösung von 11,2 g (0,12 mol) Phenol in 100 ml Dimethylformamid (DMF) wurde mit 9,9 g 30 %iger Natriummethylatlösung in Methanol (entsprechend 0,11 mol NaOMe) versetzt. Dann wurde das Methanol bei 50 °C im Vakuum vollständig abdestilliert. Zu der verbleibenden Lösung wurden 17,2 g (0,1 mol) 1-Methyl-4,5-dinitroimidazol gegeben; dann wurde 1 Std. bei 50 °C gerührt. Nach dem Abkühlen wurde mit 200 ml Wasser verdünnt; die ausgefallenen Kristalle wurden abgesaugt und aus Wasser umkristallisiert.

Ausbeute: 18,0 g (82 %) 1-Methyl-4-nitro-5-phenoxyimidazol,
Smp.: 115-118 °C.

Beispiel 26

Herstellung von 1-Methyl-4-nitro-5-dodecyloxyimidazol

Eine Lösung von 22,3 g (0,12 mol) Dodecanol in 100 ml DMF wurde mit 12,3 g (0,11 mol) Kalium-tert.-butylat versetzt. Dann wurde das tert.-Butanol bei 50 °C im Vakuum vollständig abdestilliert. Zu der verbleibenden Lösung wurden 17,2 g (0,1 mol) 1-Methyl-4,5-dinitroimidazol gegeben; dann wurde 3 Std. bei 70 °C gerührt. Danach wurde die Lösung eingedampft und der Rückstand mit heißem Petrolether extrahiert. Der Extrakt wurde eingedampft und der Rückstand aus Petrolether umkristallisiert.

Ausbeute: 11,0 g (35 %) 1-Methyl-4-nitro-5-dodecylimidazol, Smp.: 56-59 °C.

| Elementaranalyse: | | | | |
|---|---|---|---|---|
| Ber.: | C 61,7 | H 9,4 | N 13,5 | O 15,4 |
| Gef.: | C 61,6 | H 8,6 | N 13,1 | O 15,3 |

## Patentansprüche

**1.** Verfahren zur Herstellung von 4-Nitro-5-imidazolylethern und -thioethern der Formel I

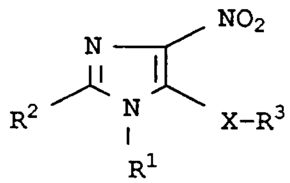

(I),

in der die Reste

- $R^1$ $C_1$- bis $C_{18}$-Alkyl, $C_2$- bis $C_8$-Alkoxyalkyl, $C_5$- bis $C_8$-Cycloalkyl, Aryl, $C_7$- bis $C_{12}$-Arylalkyl, $C_7$- bis $C_{12}$-Alkylaryl,
- $R^2$ Wasserstoff, $C_1$- bis $C_{18}$-Alkyl, $C_5$- bis $C_8$-Cycloalkyl, Aryl, $C_7$- bis $C_{12}$-Arylalkyl, $C_7$- bis Alkylaryl,
- $R^3$ $C_1$- bis $C_{18}$-Alkyl, $C_2$- bis $C_8$-Alkoxyalkyl, $C_5$- bis $C_8$-Cycloalkyl, Aryl, $C_7$- bis $C_{12}$-Arylalkyl, $C_7$- bis $C_{12}$-Alkylaryl oder 3-Pyridyl, 2-Pyridyl, 2-Pyrimldyl, 8-Chinolyl, 2-Imidazolyl, 2-Thiazolyl, 6-Purimidyl, 3-Tetrahydropyranyl und 2-Thiazolinyl, ein Gegenion des entsprechenden Alkoholates oder für den Fall, daß X = Schwefel ist $R^3$ = Wasserstoff, und
- X Sauerstoff oder Schwefel

bedeuten, wobei die genannten Aryl Reste $C_1$- bis $C_4$-Alkyl, $C_1$- bis $C_4$-Alkoxy, Halogen, Halogenalkyl

und/oder $C_1$- bis $C_4$-Alkoxymethyl, die organischen Reste $R^1$ und $R^2$ $C_1$- bis $C_4$-Alkyl, $C_1$- bis $C_4$-Alkoxy und/oder Halogen, und der Rest $R^3$ $C_1$- bis $C_6$-Alkyl, $C_1$- bis $C_6$-Halogenalkyl, $C_1$- bis $C_6$-Alkoxy, $C_1$- bis $C_6$-Dialkylamino und/oder Halogen Substituenten tragen können, dadurch gekennzeichnet, daß man Dinitroimidazole der Formel II

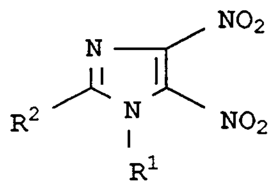

(II),

mit einem Alkohol oder Thiol der Formel III

$R^3$-XH (III),

in einem Lösungs- oder Verdünnungsmittel bei Temperaturen von 0 bis 150 °C und einem pH-Wert von 4 bis 16 umsetzt.

**2.** Verfahren nach Anspruch 1, dadurch gekennzeichnet, daß man als Lösungs- oder Verdünnungsmittel Wasser und/oder mit Wasser mischbare inerte organische Lösungsmittel verwendet.

**3.** Verfahren nach den Ansprüchen 1 und 2, dadurch gekennzeichnet, daß man als Lösungs- oder Verdünnungsmittel Wasser und/oder $C_1$-$C_4$-Alkohole verwendet.

**Claims**

**1.** A process for the preparation of a 4-nitro-5-imidazolyl ether or thioether of the formula I

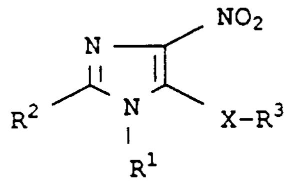

(I)

where $R^1$ is $C_1$-$C_{18}$-alkyl, $C_2$-$C_8$-alkoxyalkyl, $C_5$-$C_8$-cycloalkyl, aryl, $C_7$-$C_{12}$-arylalkyl or $C_7$-$C_{12}$-alkylaryl, $R^2$ is hydrogen, $C_1$-$C_{18}$-alkyl, $C_5$-$C_8$-cycloalkyl, aryl, $C_7$-$C_{12}$-arylalkyl or $C_7$-$C_{12}$-alkylaryl, $R^3$ is $C_1$-$C_{18}$-alkyl, $C_2$-$C_8$-alkoxyalkyl, $C_5$-$C_8$-cycloalkyl, aryl, $C_7$-$C_{12}$-arylalkyl, $C_7$-$C_{12}$-alkylaryl or 3-pyridyl, 2-pyridyl, 2-pyrimidyl, 8-chinolyl, 2-imidazolyl, 2-thiazolyl, 6-purimidyl, 3-tetrahydropyranyl or 2-thiazolinyl, or a counter-ion of the corresponding alcoholate or, if X is sulfur, $R^3$ is hydrogen, and X is oxygen or sulfur, and the stated aryl radicals may carry $C_1$-$C_4$-alkyl, $C_1$-$C_4$-alkoxy, halogen, haloalkyl, or $C_1$-$C_4$-alkoxymethyl, the organic radicals $R^1$ and $R^2$ $C_1$-$C_4$-alkyl, $C_1$-$C_4$-alkoxy or halogen and the radical $R^3$ $C_1$-$C_6$-alkyl, $C_1$-$C_6$-haloalkyl, $C_1$-$C_6$-alkoxy, $C_1$-$C_6$-dialkylamino or halogen substituents, wherein a dinitroimidazole of the formula II

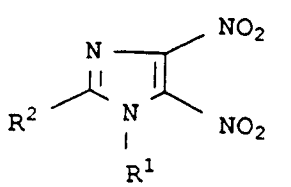

(II)

is reacted with an alcohol or thiol of the formula III

$R^3$-XH (III)

in a solvent or diluent at from 0 to 150 ° C and at a pH of from 4 to 16.

2. A process as claimed in claim 1, wherein the solvents or diluents used are water and/or water-miscible inert organic solvents.

3. A process as claimed in claim 1 or 2, wherein the solvents or diluents used are water and/or $C_1$-$C_4$-alcohols.

**Revendications**

1. Procédé de préparation d'éthers et de thioéthers de 4-nitro-5-imidazolyle de formule I

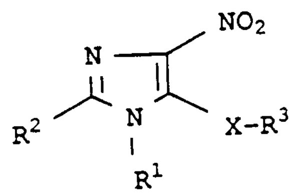

(I),

dans laquelle les restes $R^1$, $R^2$, $R^3$ et X ont les significations ci-après

- $R^1$: alkyle en $C_1$-$C_{18}$, alcoxyalkyle en $C_2$-$C_8$, cycloalkyle en $C_5$-$C_8$, aryle, arylalkyle en $C_7$-$C_{12}$, alkylaryle en $C_7$-$C_{12}$,
- $R^2$: hydrogène, alkyle en $C_1$-$C_{18}$, cycloalkyle en $C_5$-$C_8$, aryle, arylalkyle en $C_7$-$C_{12}$, alkylaryle en $C_7$-$C_{12}$,
- $R^3$: alkyle en $C_1$-$C_{18}$, alcoxyalkyle en $C_2$-$C_8$, cycloalkyle en $C_5$-$C_8$, aryle, arylalkyle en $C_7$-$C_{12}$, alkylaryle en $C_7$-$C_{12}$ ou 3-pyridyle, 2-pyridyle, 2-pyrimidyle, 8-quinolyle, 2-imidazolyle, 2-thiazolyle, 6-purimidyle, 3-tétrahydropyranyle et 2-thiazolinyle, un contre-ion de l'alcoolate correspondant ou, dans le cas où X = soufre, $R^3$ = hydrogène, et
- X: oxygène ou soufre,

lesdits restes aryle pouvant porter des substituants alkyle en $C_1$-$C_4$, alcoxy en $C_1$-$C_4$, halagéno, halogénoalkyle et/ou alcoxyméthyle en $C_1$-$C_4$, les restes organiques $R^1$ et $R^2$ pouvant porter des substituants alkyle en $C_1$-$C_4$, alcoxy en $C_1$-$C_4$ et/ou halogéno, et le reste $R^3$ pouvant porter des substituants alkyle en $C_1$-$C_6$, halogénoalkyle en $C_1$-$C_6$, alcoxy en $C_1$-$C_6$, dialkylamino en $C_1$-$C_6$ et/ou halogéno,
caractérisé en ce que l'on fait réagir des dinitroimidazoles de formule II

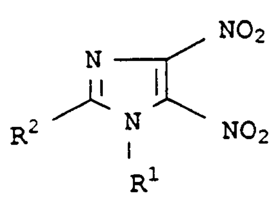

(II),

avec un alcool ou un thiol de formule III

$R^3$-XH (III),

dans un solvant ou un diluant, à des températures de 0 à 150 ° C et à un pH de 4 à 16.

2. Procédé suivant la revendication 1, caractérisé en ce que l'on utilise, comme solvants ou diluants de l'eau et/ou des solvants organiques inertes miscibles à l'eau.

3. Procédé suivant la revendication 1 ou 2, caractérisé en ce que l'on utilise, comme solvants ou diluants, de l'eau et/ou des alcools en $C_1$-$C_4$.